# EUROPEAN PATENT APPLICATION

(11) **EP 1 333 086 A1**
(43) Date of publication of application: **06.08.2003**
(21) Application number: 01974750.0
(22) Date of filing: 09.10.2001
(51) Int. Cl.: C12M 3/06

(54) **CELL CULTURE CASE**

(30) Priority: 10.10.2000 JP 2000308700
(71) Applicant: Nipro Corporation, Kita-ku, Osaka-shi, Osaka-fu, 531-8510 (JP); Scimedia Corporation, Tokyo 110-0008 (JP)
(72) Inventor: DOI, Nobutoshi, c/o NIPRO CORPORATION, Osaka-shi, Osaka 531-8510 (JP); SANO, Syushi, c/o NIPRO CORPORATION, Osaka-shi, Osaka 531-8510 (JP); TSUKIOKA, Tsuyoshi, c/o SCIMEDIA CORPORATION, Tokyo 110-0008 (JP); NOBORI, Koichi, Osaka-shi, Osaka 559-0003 (JP)
(74) Representative: Albrecht, Thomas, Dr.
(86) International application number: JP0108872
(87) International publication number: WO02031108

(57) **Abstract**

A cell culture vessel which has, sealed therein, hollow fibers having the function of a semipermeable membrane and a fibrous cell substrate being susceptible to attachment of cells, and has connecting parts communicating with the inner portion of the hollow fiber which opens at both ends of the hollow fiber and connecting parts communicating with the space between the hollow fiber and the vessel, wherein both ends of the hollow fibers are fixed to both end portions of the vessel by partitions so that the inner portion of the hollow fiber communicates with the exterior of the vessel, and wherein the cell substrate is housed in the space between the hollow fiber and the vessel; and a method for cell culture using the cell culture vessel. The cell culture vessel allows a high density culture of an animal cell, in particular, a cell prone to attach to a support.

## Description

### TECHNICAL FIELD

The present invention relates to a vessel for culturing animal cells, in particular, adherent animal cells. More particularly, the present invention relates to a cell culture vessel used for culturing adherent cells in a high density and utilizing a substance produced by the cultured cells or cultured cells per se.

### BACKGROUND ART

Heretofore, as a method for utilizing the metabolic function that cells have, there have been developed various methods utilizing cells as biological reactors, such as a method of culturing cells and utilizing a substance produced by the cells, for example, protein and a method of contacting proliferated cells with contaminating substances contained in a gas, liquid or the like to decompose the contaminating substances and thereby perform air purification or water purification. To these methods, the technology of culturing cells in a high density is indispensable and therefore various high density cell culture methods have been developed and utilized.

General cell culture methods include a stationary culture method in which cells are cultured in a stationary state in a vessel such as a glass dish. However, such method involving use of a dish or other vessels is limited in its application and remains to be utilized only on an experimental scale since a vessel having a large surface area and a large volume is required for culturing a large quantity of cells.

To make up for the defects of stationary culture methods, a spinner culture method has been developed in which cells are cultured in a large volume tank while stirring the cells by means of a stirrer. The application of this method is expanding from an experimental level to an industrial level. However, in such spinner culture methods involving use of a tank or other vessels, proliferation reaches a plateau at a cell culture density on the order of from 1×10⁶ to 5×10⁶ cells/ml and the cell culture density will no longer increase. As a result, to increase the concentration of the produced substance to a necessary concentration, a cumbersome operation, such as concentration is necessary and further, in order to secure a necessary amount of cells, it may be sometimes the case that a vessel having a very large volume is required and therefore many difficulties are encountered in their industrial application.

Also, the spinner culture method is of a form suitable for culturing buoyant cells but is not suitable for high-density culture of adherent cells. However, along with development of genetic recombination technology in recent years, various valuable substances have come to be produced by utilizing cells that are artificially modified so as to produce specified substances, for example, recombinant cells or fused cells. These cells include adherent cells and buoyant cells. Adherent cells derived from animal tissues are utilized in comparatively many cases.

Accordingly, as a method of applying a spinner culture method to adherent cells, a method that involves changing glass beads in a tank as a carrier for adherent cells has been conceived. However, to make the surface area of the beads greater, a tank having a larger volume is required. Even in that case, a limit exists at a cell culture density on the order of 5×10⁶ cells/ml, so that it is impossible to increase culturing efficiency. Further, necessity of a large scale facility results in a high cost. Therefore, a method that enables high-density cell culture in a small space has been demanded.

As a method that enables high-density cell culture in a small space, a cell culture technology that utilizes hollow fibers has received attention. This method includes culturing cells in a vessel that encloses therein hollow fibers having a void in the central portion thereof with respect to the diameter. This is a rational method in that the vessel, in which a number of thin hollow fibers are contained, has an advantage that a large cell attachment area can be obtained inside a limited space and in addition, use as an adherend for cells of a material such as hollow fibers having permeability to a substance enables supply of nutrients necessary for the proliferation of cells through the hollow fibers. An example of a culture method involving use of such hollow fibers has been introduced by Knazak et al. as a method including supplying necessary nutrients to cells through hollow fibers contained in a vessel while at the same time letting the cells adhere to the hollow fibers per se, thereby ensuring anchorage needed for the proliferation of adherent cells (JP 49-41579 A). Further, as advanced versions of the method including use of hollow fibers, a method in which a cellulose acetate fiber is used as a hollow fiber (JP 54-6634 B), a method including use of a polysulfone fiber as a hollow fiber (JP 62-130678 A), a method including use of a polyolefin fiber as a hollow fiber (JP 63-17685 A) and the like have been proposed. In addition, as a method of improving the cell adherence of the hollow fibers and further increasing the proliferation efficiency, methods including use of a porous membrane made of polyolefin (JP 2-16972 A, JP 3-292884 A, JP 3-292885, etc.) and the like have been proposed.

However, even when culture is performed by actually using adherent cells to culture them by a culture method that allows the cells to adhere to the various kinds of hollow fibers per se, cells will not proliferate as is expected. This is because the hollow fiber per se is given the role of providing an anchoring means to which cells adhere and of exchanging nutrients and hence accordingly as the cells adhere on the surface of the hollow fibers, results similar to the case where the surface of the hollow fibers are clogged are obtained by the cells that are adhered thereon.

Further, the nutrients can be supplied to only the cells that adhere thereto, so that accordingly as the cells proliferate, the area where exchange of nutrients is possible is decreased, with a concomitant decrease in efficiency of nutrients exchange of the hollow fibers.

For this reason, in the case where culture of the adherent cells is intended in a higher cell density, it is insufficient to just allow the adherent cells to adhere on the surface of the hollow fibers and it is necessary to make the cells proliferate three-dimensionally in interstices between the hollow fibers and the vessel in order to maintain the efficiency of nutrients exchange of the hollow fibers. In the case of the above-mentioned method of allowing the cells to adhere on the surface of the hollow fibers, it is possible to supply nutrients to those cells that adhere two-dimensionally on the surface of the hollow fibers. However, it cannot be said to be suitable for the case where a three-dimensional high cell density culture as high as 1×10⁸ cells/ml or more is intended.

Methods have been proposed which are intended to improve the method of adhering cells to hollow fibers as described above so that high cell density culture of cells is performed three-dimensionally. One of them is a method in which use is made of two tubes supplying necessary nutrients and gas and a sheet-like 3D matrix spirally wound around the two tubes as a carrier for adhering the cells, one of the tubes being for gas exchange and the other for nutrient exchange (BP 2178447 B). Also, in order to increase the culture efficiency of the above-mentioned technology so that it can be applied to a bioartificial liver there has been proposed a device similar to the above-mentioned technology, which includes a cylindrical vessel containing a sheet-like 3D matrix on to which hollow fibers are attached in order at intervals of 0.5 to 5 mm (JP 11-514229 A).

The above-mentioned proposals concern methods of obviating the defects of the hollow fiber type cell culture methods that cause a decrease in the efficiency of proliferation due to the adherence of cells on the surface of the hollow fiber as described hitherto by use of, besides the hollow fibers, a carrier allowing cells to adhere thereto, and preventing a decrease in the efficiency of the hollow fibers.

However, the above-mentioned methods proposed in BP 2178477 B and JP 11-514229 A are not suitable enough for actually culturing cells in high densities. This is because the method described in BP 2178477 B uses a single conduit as a tube for use in exchanging oxygen, nutrients and the like and hence in actuality it will be impossible to supply sufficient oxygen, nutrients and the like to all the cells that are cultured in a high density. As a result, a cell culture efficiency or high cell culture density, which makes the disclosed device useful as a biological reactor, will not be obtained.

Also, in the method described in JP 11-514229 A, improvement has been focused on the supply of nutrients and the exchange of high concentration oxygen. In this case too, it can not be said that sufficient performance was exhibited. The reason for this is that according to JP 11-514229 A, the feature is in the arrangement of the hollow fibers in a state where they are attached to the surface of a sheet-like 3D matrix that serves as a carrier for adhering cells. In this case, of the portions of the surface of the hollow fibers used for the exchange of gases and nutrients, the portion that is attached to the sheet cannot effectively perform exchange of substances, which results in a decrease in the efficiency of exchange of substances corresponding to the area of the attached portion.

Further, the fact that the carrier for adhering cells is in the form of a sheet has the following problem. That is, since cells proliferate as they adhere on the surface of the adhering carrier, the rate of increase of cells, that is, culture density, is proportional to the surface area thereof. Therefore, there is a limitation in increasing the surface area of the sheet-like adhering carrier within a vessel having a volume limited to a prescribed volume and hence the obtained cell density is limited so that the carrier for adhering cells which is in the form of a sheet is a form unsuitable for a high density culture.

Also, according to the above-mentioned patent publications, for application to a bioartificial liver, the cell number was increased so that the cells were proliferated to a culture density of 20×10⁵ cells/ml and reference was also made to the possibility of a culture density of 220×10⁵ cells/ml. Although such culture density may possibly make enough sense in the case where it is used for a human body for the purpose of saving a life, high density culture as high as 1×10⁸ cells/ml or more is necessary for providing a general biological reactor used for the purpose of industrially utilizing the substance produced by the cells and the above-mentioned technology could not be said to be sufficient.

Namely, under the circumstances, the present invention has been accomplished and an object of the present invention is to provide a method of culturing animal cells, in particular adherent cells, in a high density. Specifically, an object of the present invention is to provide a hollow fiber culture vessel that includes therein a carrier capable of adhering cells thereto as a cell substrate so that the best performance can be obtained from the hollow fibers with respect to the efficiency of exchange of substances and that enables culturing adherent animal cells at a high density for a long period of time.

### DISCLOSURE OF THE INVENTION

The inventors of the present invention have found that culturing at a density much higher than that conventionally attained is possible by performing cell culture at a high density by use of a cell culture vessel containing a fibrous cell substrate to which cells are prone to adhere and hollow fibers capable of maintaining conditions necessary for the proliferation of cells, thereby achieving the present invention.

Therefore, the present invention relates to a cell culture vessel and a cell culture method as described below.
1. A cell culture vessel including enclosed therein hollow fibers having a function of a semipermeable membrane and a fibrous cell substrate to which cells are prone to adhere.
2. A cell culture vessel according to item 1, in which the hollow fibers are open at both ends thereof and further independently including a connection port communicating with lumens of the hollow fibers and a connection port communicating with interstice portions between the vessel and the hollow fibers.
3. A cell culture vessel according to item 1 or 2, in which the cell substrate is housed in the interstice portions between the hollow fibers and the vessel.
4. A cell culture vessel according to any one of items 1 to 3, in which both ends of the hollow fibers are fixed to both end portions of the vessel through partitions.
5. A cell culture vessel according to any one of items 1 to 4, in which the cell substrate is a fiber assembly fixed at both ends thereof to both end portions of the vessel through a sealing material.
6. A cell culture vessel according to any one of items 1 to 5, in which the cell substrate is at least one fiber assembly (fiber bundle).
7. A cell culture vessel according to item 6, in which the cell substrate is a fiber assembly including fibers each having a diameter of 0.01 to 1,000 µm.
8. A cell culture vessel according to any one of items 1 to 7, in which the cell substrate is a fiber assembly made of organic fibers or inorganic fibers.
9. A cell culture vessel according to item 8, in which the organic fibers are fibers selected from the group consisting of polyolefin fibers, polyester fibers, polystyrene fibers, polycarbonate fibers and polyamide fibers.
10. A cell culture vessel according to item 9, in which the inorganic fibers are ceramic fibers or glass fibers.
11. A cell culture vessel according to any one of items 1 to 10, in which the hollow fibers have an inner diameter of 10 to 1,000 µm, a membrane thickness of 1 to 500 µm, and a fractionation molecular weight of 1,000 to 100,000.
12. A cell culture vessel according to item 11, in which the cell substrate is at least one fiber assembly and a ratio of the cell substrate to the hollow fibers is 1 ≤ χ/n ≤ 100, where n is the number of fiber assemblies and χ is the number of hollow fibers.
13. A cell culture vessel according to item 12, in which the cell substrate is a fiber assembly and the fiber assembly and the hollow fibers are dispersed uniformly in the cell culture vessel.
14. A cell culture vessel according to item 13, in which the cell substrate is a fiber assembly and the fiber assembly is inserted into the interstices of the hollow fibers.
15. A cell culture vessel according to any one of items 12 to 14, in which a total volume of the cell substrate and the hollow fibers occupies a volume of at least 20% of that of the whole cell culture vessel.
16. A cell culture vessel according to any one of items 12 to 15, in which a total volume of the cell substrate and the hollow fibers occupies a volume of 20 to 85% of that of the whole cell culture vessel.
17. A cell culture vessel according to any one of items 1 to 16, in which the cell culture vessel is a cylindrical vessel that has a vessel end portion connection port communicating with lumens of the hollow fibers on each end portion thereof and two vessel side connection ports communicating with the cell substrate and interstice portions between the hollow fibers and the cell culture vessel, respectively, on a side of the vessel, and in which an end portion chamber and a partition are provided at both ends of the inside of the vessel.
18. A cell culture vessel according to item 17, in which cells are adhered to the cell substrate, a culture medium is supplied from outside communicating with the hollow fibers and replenishment of nutrients and recovery of cell metabolism waste products are performed by a semipermeable membrane function of the hollow fibers.
19. A cell culture vessel according to item 18, in which oxygen is circulated.
20. A cell culture vessel according to any one of items 1 to 19, in which the cells are adherent cells derived from an animal.
21. A cell culture method, characterized by proliferating cells by using a cell culture vessel according to item 20.

In the present invention, the vessel for enclosing the hollow fibers and the cell substrate may be any material as long as the vessel per se has no toxicity to cells and gives no adverse influences on the cells by denaturation, decomposition, etc. as a result of sterilization, washing or upon its contact with a culture liquid, or similar operation which is necessary when it is used in culture, and can enclose and contain the hollow fibers and the cell substrate. For example, there may be employed any polymeric material such as polycarbonates, polystyrenes, acrylic resins, and polyolefin resins. There may also be employed metallic materials including iron, aluminum, etc., or inorganic materials such as glass and ceramics.

The shape of the vessel for enclosing the hollow fibers and the cell substrate may be any form as long as it can hold the hollow fibers and the cell substrate as enclosed therein. For example, it includes a cylindrical shape, a box-like shape, a bag-like shape, a spherical shape, and the like. The shape of the vessel may be designed in various forms depending on the purpose as appropriate. Generally, the vessel is a cylindrical vessel. Preferably, such vessel is provided with a vessel end connection port for introducing or discharging a medium or gases necessary for the cell culture and a connection port capable of introducing or discharging cells or a substance that the cells produce, i.e., a vessel side connection port.

The hollow fibers as used herein are fibers having a void in the central part thereof. Any material may be used therefor as long as it is a hollow fiber that has the following properties.

That is, the fiber per se has no toxicity to cells and gives no adverse influences on the cells by denaturation, decomposition, etc. upon its contact with a sterilization, washing or culture liquid, or similar operation which is necessary when it is used in a culture and it has the property of a semipermeable membrane such that it is not permeated by a protein-like substance that the cells produce but has permeability to substances having smaller molecular weights than the protein-like substance. Examples of the material include organic materials such as cellulose resins, polyolefins, fluoro resins, polysulfones, polyether sulfones, polycarbonates, and acrylic resins as well as inorganic materials such as ceramics. The material may also include various surface treated membranes such as those treated by a chemical treatment or surface modification as long as it is a hollow fiber that satisfies the above-mentioned properties.

The form of the hollow fibers may be designed to be in any appropriate form with respect to the inner diameter, outer diameter, thickness, total length and the like parameters of the hollow fiber depending on the cells used, necessary amount of the substance that the cells produce, period of culture, scale of the facility and the like. Specifically, the above-mentioned hollow fiber has an inner diameter of 10 to 1,000 µm, preferably 50 to 500 µm, a membrane thickness of 1 to 500 µm, preferably 5 to 100 µm, and a fractionation molecular weight of 1,ₒ000 to 100,000, preferably 5,000 to 50,000. Usually, the cell culture vessel encloses 100 to 40,000 hollow fibers and preferably 500 to 20,000 hollow fibers therein.

The mode of housing the hollow fibers in the vessel may be any mode as far as it is a method in which the lumen portions of the hollow fibers communicate with the outside of the above-mentioned vessel and ends of the hollow fibers can be fixed to both ends of the vessel through partitions. The sum of the volumes of the hollow fibers and of the cell substrate in the whole cell culture vessel occupies at least 20%, preferably 20 to 85%, more preferably 25 to 75%, and particularly preferably 35 to 65%, of the volume of the vessel. It is preferred that the shape of the vessel be designed in accordance with this filling ratio.

In the present invention the cell substrate is a carrier for adherent cells to proliferate thereon. Its material preferably is a fiber that is not a hollow fiber. Its material may be any substance as far as the support per se has no toxicity to cells and gives no adverse influences on the cells by denaturation, decomposition, etc. as a result of sterilization, washing or upon its contact with a culture liquid, or a similar operation which is necessary when it is used in a culture and it is a material that is easy for cells to adhere thereto. For example, a group consisting of polymeric materials includes polyolefin resins, polyester resins, polystyrene resins, polycarbonates, polyamide resins, and the like. Also, inorganic materials include ceramics, glass, and the like. Polyester resins, glass and the like are preferable. Such cell substrates may be subjected to a physical or chemical treatment on their surface in order to increase the adherence of cells.

The form of the cell substrate may be a form of a monofilament or a fiber bundle made of a set of monofilaments. The fiber diameter of a monofilament is in compliance with the size of the cells that are the object of culture and it is preferred for the proliferation of the cell that the monofilament have an outer diameter equivalent to or greater than that of the cells. The outer diameter of the fiber suitable for the cells is 0.01 to 1,000 µm, preferably 0.05 µm to 200 µm, more preferably 0.1 to 200 µm. The fiber length is selected depending on the length of the vessel. When these monofilaments are combined to make a fiber bundle, the fiber bundle is preferably a set of 2 to 500, more preferably 2 to 100, of the monofilaments described above.

In the case where the cell substrate is in the form of a fiber bundle made of a set of a plurality of monofilaments, the form of the fiber bundle may take any form such as a twisted yarn, nonwoven fabric, or knitted yarn. A form of nonwoven fabric is preferable that has interstices between filaments constituting the fiber bundle and that allows cells to ingress and adhere thereto. Preferably, the interstices between the fiber bundles are adjusted depending on the size of the cells that are the object of the culture; the interstices are preferably 0.01 to 500 µm and more preferably 0.1 to 200 µm. In the case of a fiber bundle, it is preferred that interstices are uniformly provided between filaments but the fiber bundle may take a form in which the filaments are partially in contact with each other, such as a nonwoven fabric, twisted yarn, knitted yarn or the like.

With respect to the form of housing and enclosing of the above-mentioned fibrous cell substrate in the vessel, it is preferable to house them in the inner space of the vessel in parallel with the interstices of the hollow fibers. However, the cell substrate may be positioned in a slightly oblique state with respect to the hollow fibers or it may be positioned so that it is in a serpentine state between the hollow fibers. The ends of the fibrous cell substrate are fixed to both ends of the cell culture vessel through partitions. The number of the fibrous cell substrates to be enclosed may be increased or decreased as appropriate depending on the objective culture conditions and they can be housed in any number depending on the size of the vessel. However, it is preferred that they be housed in a ratio of 1 ≤ χ/n ≤ 100 where n is the number of units of fiber bundles, a unit being a set of 1 to 500 monofilaments, and χ is the number of hollow fibers. More preferred is the case where 1 ≤ χ/n ≤ 50.

In the present invention, the animal adherent cells collectively mean those cells that are derived from an animal tissue and that have properties of proliferating as they adhere to an anchorage of some sort upon their proliferation and the cells may be used without being particularly limited with respect to kind. Examples thereof include cells derived from the ovary of a Chinese hamster (CHO cells), cells derived from the kidney of an African green monkey (Vero cells), cells derived from human fetus (MRC-5 cells), cells derived from the kidney of a newborn Syrian hamster (BHK cells), cells derived from human uterocervical cancer (Hela cells), cells derived from murine fetus (Balb3T3 cells), and cells obtained by introducing an exogenous gene into these naturally occurring cells.

Although the present invention has been achieved in order to provide a culture vessel suitable for adherent cells, it is obvious that in the case where buoyant cells are cultured, the present invention also will exhibit a higher culture efficiency than that of a biological reactor with only hollow fibers intended to culture buoyant cells, since the cell substrate enclosed as a cell adhering carrier ensures spaces in the interstices between the hollow fibers where the buoyant cells can exist and is arranged so that the efficiency of exchange of nutrients can be maximized. Therefore, the culture vessel in accordance with the present invention is not limited to the culture of adherent cells.

### BRIEF DESCRIPTION OF THE DRAWINGS

Fig. 1 is a cross-sectional view showing a cell culture vessel according to the present invention; and
Fig. 2 is a circuit diagram of a cell culture system according to one embodiment of the present invention.

### BEST MODE FOR CARRYING OUT THE INVENTION

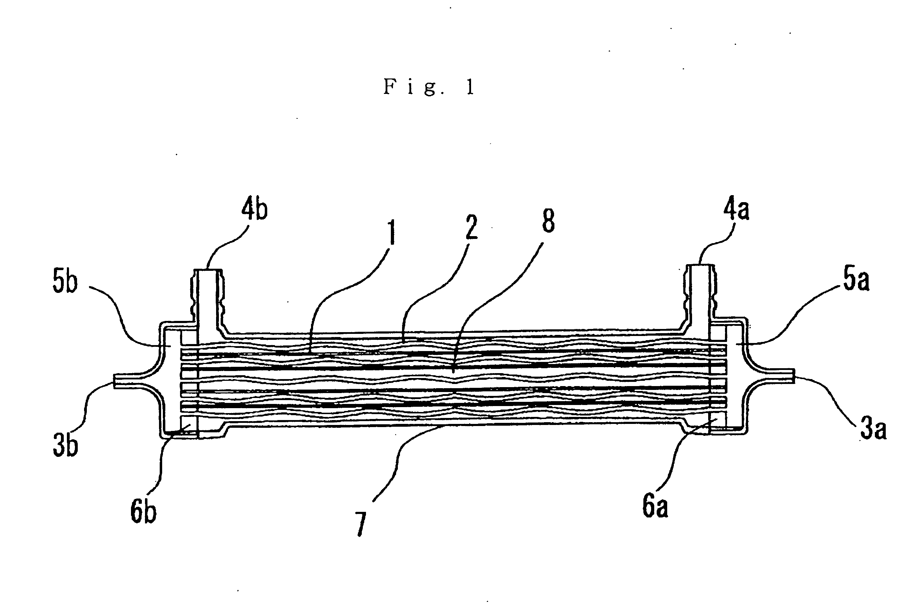

Fig. 1 is a schematic diagram illustrating a specific embodiment of the vessel of the present invention. Cell substrates 1 are inserted in parallel to a number of hollow fibers 2. The cell substrates 1 and hollow fibers 2 are fixed through partitions 6a and 6b that are sealants to both end portions of a vessel 7, respectively. Inner lumens of the hollow fibers are integrated into one at end chambers 5a and 5b at both ends and communicate with the outside through connection ports 3a and 3b on both ends of the vessel. As for nutrients necessary for culturing cells, necessary nutrients are supplied through the hollow fiber membranes to a cell proliferation space 8 surrounded by the outsides of the hollow fibers and the vessel by supplying a culture medium to the lumens of the hollow fibers through a circuit connected to the connection ports 3a and 3b. On the contrary, waste products metabolized by the cells are incorporated into the insides of the hollow fibers from the cell proliferation space 8. The cell proliferation space 8 is connected to the outside circuit through connection ports 4a and 4b opened on a side of the vessel 7. The connection ports 4a and 4b are used for injecting cells, replenishing the culture medium necessary for the proliferation of the cells, taking out substances that the cells produce and so on as appropriate.

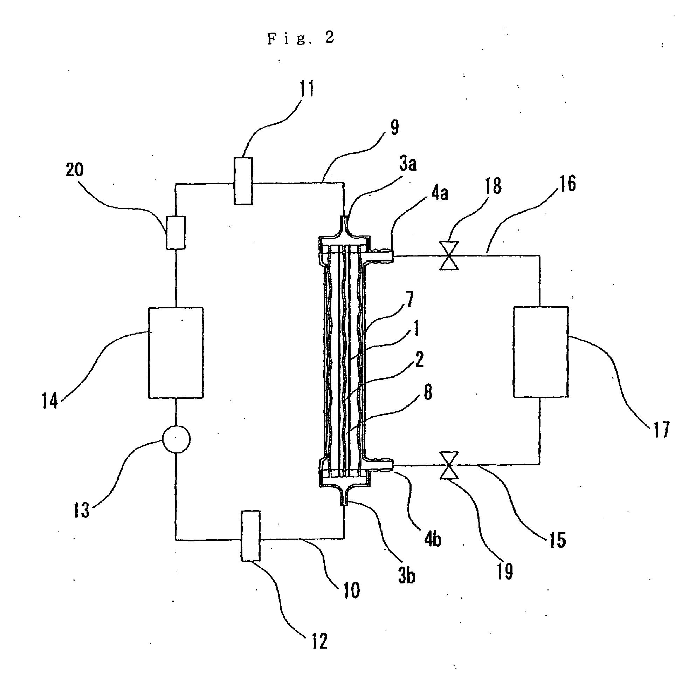

Fig. 2 shows an example of a cell culture circuit using the cell culture vessel of the present invention. The lumens of the hollow fibers are connected to end portion connection port connection circuits 9 and 10 through connection ports 3a and 3b at both ends of the cell culture vessel 7. In circuit 9 are connected an oxygen densitometer 11 that monitors the concentration of oxygen in the culture liquid supplied and a gas exchanger 20 for adjusting the concentration of a gas in the culture liquid circulating in the circuit. In circuit 10 is connected a pH meter 12 that monitors the pH of the culture medium supplied. The circuits 9 and 10 are connected to a culture liquid tank 14 and a pump 13 supplies the culture liquid. It is preferred that the components of the culture liquid be appropriately selected depending on the kind of cells. The concentration of oxygen and pH of the culture liquid supplied to the vessel 7 are monitored by the meters arranged, respectively, so that they maintain an optimal environment as appropriate. The cell culture space 8 is connected to an outer buffer tank 17 through the connection ports 4a and 4b on the side of the vessel 7 by the vessel side connection port connection circuits 15 and 16. In the midway of the circuits are provided valves 18 and 19 and the cell culture space 8 is made to be a closed system so that the culture liquid cannot be localized to either one of the inside or outside of the hollow fibers as a result of filtration occurring due to a difference in pressure between the inside and outside of the hollow fibers. In the case where the substance that the cells produce is taken out, the valves are opened and the product is collected from the buffer tank. The amount of liquid decreased is replenished to the cell culture space as appropriate through the hollow fiber membrane, so that the amount of the liquid existing in the cell culture space is maintained constant.

The cell culture method of the present invention is performed by adhering cells to the cell substrate 1 existing in interstice portions of the above-mentioned hollow fibers 2 and the above-mentioned cell culture vessel 7, supplying a culture medium through the vessel end portion connection ports 3a and 3b provided on both end portions of the vessel, and optionally circulating oxygen to proliferate the above-mentioned cells. The culture medium may be any one as far as it is useful for culturing animal adherent cells. For example, a liquid culture medium (90% RPMI-1640 and 10% calf fetal serum, manufactured by LTI) is exemplified. The culture conditions are in accordance with ordinary ones.

### EXAMPLE

Hereinafter, the present invention will be described and exemplified by an Example. The Example is one example of a culture in which the cell culture vessel of the present invention is used and the constitution of the circuit to be connected, form of the circuit, the culture liquid circulating method, the mode of supplying the culture liquid, the adjustment of the culture environment and so on may be varied as appropriate by one skilled in the art and the present invention should not be considered to be limited by the Example.

### Example 1

About 4,500 hollow fibers made of cellulose acetate having an inner diameter of 200 µm and a membrane thickness of 15 µm (having a total inner surface area of the hollow fibers of about 0.5 m²) were enclosed in a cylindrical vessel made of polycarbonate (200 mm in length and 30 mm in diameter). Also, as a cell substrate, fiber bundles made of a bundle of 30 polyethylene terephthalate (PET) fibers having a fiber diameter of 20 µm (190 mm in length) were enclosed in the vessel in a ratio of hollow fibers: PET fiber bundles = 24:1 so that the fiber bundles were uniformly dispersed between the hollow fibers. The ends of the hollow fibers and of the cell substrates were fixed to both ends of the cylindrical vessel by using urethane resin. The fractionation molecular weight of the hollow fiber was in the vicinity of about 20,000 and the volume that the hollow fiber and the cell substrate occupied was adjusted to be about 38% based on the inner volume of the vessel.

By using the cell culture vessel shown in Fig. 1, the cell culture circuit shown in Fig. 2 was assembled and the whole circuit was sterilized by irradiation with γ rays. In the culture liquid tank 14 was charged 10 L of a liquid culture medium (90% RPMI-1640 and 10% calf fetal serum, manufactured by LTI) and this was circulated at a flow rate of 5 to 10 mL/minute while appropriately adjusting the concentration of oxygen to be maintained at 5 ppm in the lumens of the hollow fibers 2 and the connection ports 3, 9, and 10 thereof. The liquid culture medium to be circulated was adjusted to have a carbon dioxide concentration of about 5% by the gas exchanger 20. In the circuitry made up by the culture space 8 of the cell culture vessel, the circuits 15 and 16, as well as the buffer tank 17 was filled a liquid culture medium having the same composition as that of the above-mentioned liquid culture medium and cells derived from human liver (Hep-G2 cells) were injected therein in an amount of 1×10⁵ cells/mL and cultured for 60 days.

As a result of culturing for 60 days, the number of cells reached 5.0×10⁸ cells/mL. As compared with the conventional method which was said to attain about 1 to 5×10⁷ cells/mL, a high density as high as about 10 times was obtained.

### INDUSTRIAL APPLICABILITY

Use of the cell culture vessel of the present invention makes it possible to culture animal adherent cells efficiently at a high density to maintain a high substance productivity. That is, in the case where production of a culture product is performed in the same amount, the facility can be reduced to a scale about 1/10 as large as the conventional facility, and use of the facility on the same scale results in a production amount of about 10 times as large as that conventionally obtained.

## Claims

1. A cell culture vessel comprising enclosed therein hollow fibers having a function of a semipermeable membrane and a fibrous cell substrate to which cells are prone to adhere.

2. A cell culture vessel according to claim 1, wherein the hollow fibers are open at both ends thereof and further independently comprising a connection port communicating with lumens of the hollow fibers and a connection port communicating with interstice portions between the vessel and the hollow fibers.

3. A cell culture vessel according to claim 1 or 2, wherein the cell substrate is housed in the interstice portions between the hollow fibers and the vessel.

4. A cell culture vessel according to any one of claims 1 to 3, wherein the ends of the hollow fibers are fixed to both end portions of the vessel through partitions.

5. A cell culture vessel according to any one of claims 1 to 4, wherein the cell substrate is a fiber assembly fixed at ends thereof to both end portions of the vessel through a sealing material.

6. A cell culture vessel according to any one of claims 1 to 5, wherein the cell substrate is at least one fiber assembly (fiber bundle).

7. A cell culture vessel according to claim 6, wherein the cell substrate is a fiber assembly comprising fibers each having a diameter of 0.01 to 1,000 µm.

8. A cell culture vessel according to any one of claims 1 to 7, wherein the cell substrate is a fiber assembly made of organic fibers or inorganic fibers.

9. A cell culture vessel according to claim 8, wherein the organic fibers are fibers selected from the group consisting of polyolefin fibers, polyester fibers, polystyrene fibers, polycarbonate fibers and polyamide fibers.

10. A cell culture vessel according to claim 9, wherein the inorganic fibers are ceramic fibers or glass fibers.

11. A cell culture vessel according to any one of claims 1 to 10, wherein the hollow fibers have an inner diameter of 10 to 1,000 µm, a membrane thickness of 1 to 500 µm, and a fractionation molecular weight of 1,000 to 100,000.

12. A cell culture vessel according to claim 11, wherein the cell substrate is at least one fiber assembly and a ratio of the cell substrate to the hollow fibers is 1 ≤ χ/n ≤ 100, where n is the number of fiber assemblies and χ is the number of hollow fibers.

13. A cell culture vessel according to claim 12, wherein the cell substrate is a fiber assembly and the fiber assembly and the hollow fibers are dispersed uniformly in the cell culture vessel.

14. A cell culture vessel according to claim 13, wherein the cell substrate is a fiber assembly and the fiber assembly is inserted into the interstices of the hollow fibers.

15. A cell culture vessel according to any one of claims 12 to 14, wherein a total volume of the cell substrate and the hollow fibers occupies a volume of at least 20% of that of the whole cell culture vessel.

16. A cell culture vessel according to any one of claims 12 to 15, wherein a total volume of the cell substrate and the hollow fibers occupies a volume of 20 to 85% of that of the whole cell culture vessel.

17. A cell culture vessel according to any one of claims 1 to 16, wherein the cell culture vessel is a cylindrical vessel that has a vessel end portion connection port communicating with lumens of the hollow fibers on each end portion thereof and two vessel side connection ports communicating with the cell substrate and interstice portions between the hollow fibers and the cell culture vessel, respectively, on a side of the vessel, and in which an end portion chamber and a partition are provided at both ends of the inside of the vessel.

18. A cell culture vessel according to claim 17, wherein cells are adhered to the cell substrate, a culture medium is supplied from outside communicating with the hollow fibers and replenishment of nutrients and recovery of cell metabolism waste products are performed by a semipermeable membrane function of the hollow fibers.

19. A cell culture vessel according to claim 18, wherein oxygen is circulated.

20. A cell culture vessel according to any one of claims 1 to 19, wherein the cells are adherent cells derived from an animal.

21. A cell culture method, **characterized by** proliferating cells by using a cell culture vessel according to claim 20.

22. A method of producing a cell product, comprising using a cell culture method according to claim 21.
